# EUROPEAN PATENT SPECIFICATION

(11) **EP 1 693 051 B1**
(45) Date of publication and mention of the grant of the patent: **04.06.2008**
(21) Application number: 05003405.7
(22) Date of filing: 17.02.2005
(51) Int. Cl.: A61K 9/00

(54) **Composition containing chitosan for sustained drug release**
Zubereitung zur kontinuierlichen Arzneimittelfreisetzung Chitosan enthaltend
Composition pour libération soutenue comprenant du chitosan

(43) Date of publication of application: 23.08.2006
(73) Proprietor: The Jordanian Pharmaceutical Manufacturing Co., 11710 Naor (JO)
(72) Inventor: Badwan, Adnan Dr., 11185 Amman (JO); Al-Remawi, Mayyas Mohammad Ahmad Dr., 11710 Naor (JO); Mohammed, Murshed Abdel-Qader Murshed, 11118 Amman (JO)
(74) Representative: Winkler, Andreas Fritz Ernst

(56) References cited:
- EP-A- 0 343 306
- WO-A-95/22315
- WO-A-20/04069230
- ACIKGOZ M ET AL: "CHITOSAN MICROSPHERES OF DICLOFENAC SODIUM, II: IN VITRO AND IN VIVO EVALUATION" PHARMAZIE, DIE, PHARMAZEUTISCHER VERL., ESCHBORN, DE, vol. 50, no. 4, 1 April 1995 (1995-04-01), pages 275-277, XP000494763 ISSN: 0031-7144
- DATABASE WPI Section Ch, Week 199404 Derwent Publications Ltd., London, GB; Class B07, AN 1994-031721 XP002336182 & JP 05 339149 A (TAISHO PHARM CO LTD) 21 December 1993 (1993-12-21)
- DATABASE WPI Section Ch, Week 200501 Derwent Publications Ltd., London, GB; Class C03, AN 2003-817546 XP002336212 & JP 03 605613 B2 (KANSAI KITOSAN YG) 22 December 2004 (2004-12-22)

## Description

Chitosan is a natural substance that is highly available in nature. It is inexpensive, non-toxic, biodegradable, and biocompatible when compared with other polymers. Pharmaceutical uses of chitosan are very numerous. The scientific and medical literature lists hundreds of industrial, medical and dietary applications for chitosan, see J. Karlsen and O. Skaugrud, Excipient Properties of Chitosan, Manufacturing Chemist, 62, p. 18, 1991, and I. Orienti, K. Aieda, C. Ponti, E. Gianasi, V. Zecchi, Progesterone Loaded Chitosan Microspheres, Effect of triethylene glycol glutarate linked to the chitosan molecule on drug release, S.T.P. Pharm. Sci., 6, p. 424-429, 1996. Chitosan is considered to be non-digestible by humans when taken by oral route due to lack of chitosanases, which are present, however, in some bacteria.

As a drug delivery vehicle, chitosan is considered as the drug carrier for the 21^{st} century. It has been examined extensively by the pharmaceutical industry for its potential in controlled drug delivery systems. The use of chitosan in controlled drug delivery systems aims to prepare solid dosage forms including microparticles or macromolecular systems kinetically controlling drug release in order to make the release more dependent on the pharmaceutical formulation than the physicochemical characteristics of the drug. Examples are as follows: chitosan direct compression tablets, chitosan microspheres that adhere to stomach wall, chitosan cross-linked with acetic anhydride and glutaraldehyde forms a spongy structure upon exposure to dissolution medium, and chitosan beads prepared as controlled release drug carrier.

The administering of solid drug compositions is sometimes not convenient for some patients. Solid dosage forms can not be given to children, babies and newborns, there is a difficulty in swallowing solid dosage forms compared to liquid forms. Further, there are certain adults that can not swallow tablets and, additionally drug dose can be given in larger amount in liquid dosage forms compared to solid dosage forms, especially for drugs of high strength.

WO 95/22315 A discloses a liquid ophthalmic sustained release delivery system of a pH between 3.0 and 6.2, comprising chitosan and an active agent. The system is applied to the eye wherein upon contact with the higher pH of the ocular fluid said liquid system is converted to a stiff gel.

M. Açikgoz et al.: "Chitosan Microspheres of Diclofenac Sodium, II: In Vitro and in Vivo Evaluation" Die Pharmazie, Pharmazeutischer Verlag, Eschbom, DE, vol. 50, No. 4, 1 April 1995, pages 275-277, describes a biodegradable diclofenac sodium solid microsphere system using chitosan, wherein the desired drug release profile is obtained by an appropriate selection of the particle size distribution.

WO 2004/069230 A discloses a pharmaceutical composition comprising an active agent and chitosan for sustained drug release or muco-adhesion. The chitosan utilized is either soluble in diluted aqueous HCl or is fully water-soluble.

JP 05-339149 A discloses a release-controlled suspension containing cross-linked chitosan and acidic drug in the form of chitosan microspheres.

It is therefore the object of the present invention to overcome the disadvantages of the prior art and to provide use of a chitosan drug composition for sustained drug release, which may be easily and reliable administered to a patient.

This object is achieved by a use of a composition for preparation of a liquid sustained release delivery system for oral administration, wherein the composition comprises : a) chitosan dissolved in a solvent having a pH of below about 4.0, wherein the chitosan is selected from the group of chitosans precipitating in a pH range of between 5.0 and about 7.5 and b) at least one drug compound soluble in the chitosan solution.

It is preferable that the chitosan is selected from the group of chitosans precipitating in a pH range of between 6.0 to 7.5, more preferably 6.5 to 7.5, and most preferably 7.0 and 7.5.

More preferably, the solvent is 0.1 M HCl and/or water.

In one preferred embodiment, the drug is diclofenac, ibuprofen sodium, valproate sodium and/or the like.

Preferably, the drug is present in the liquid composition in an amount of 0.1 - 10 % w/v, more preferably 0.1 - 2 % w/v.

Also preferably, chitosan is present in the liquid composition in an amount of 1 - 15 % w/v, more preferably 1 - 10 % w/v, most preferably 1 - 5 % w/v.

It was surprisingly found that using the liquid composition according to the present invention the stomach may be protected from the harsh side effect of acidic drugs, such as diclofenac sodium or ibuprofen sodium, since the release of the drug is mainly sustained to the intestinal tract, wherein the pH is somewhat higher. Up to now, diclofenac sodium or ibuprofen sodium seemed to be insoluble in acidic media. However, when used in the liquid composition according to the present invention it turns to be soluble in acidic media (pH <5.0), if it is combined with a respective chitosan polymer solution.

With increasing pH the drug released slowly due to chitosan precipitation according to the pH value. The pH of the human digestive tract varies between 1.0-7.8, wherein the stomach being acidic (pH around 1.0-3.0) and the small intestine having a pH of about 5.50-7.8.

Using the inventive liquid composition, the release of a drug may be targeted to the specific regions in the gastrointestinal tract, for example duodenum and colon. It is assumed that the sustained release depends, amongst others, on the molecular weight of chitosan used with the drug.

Further, it was observed that an improvement in drug resident time in the stomach can be obtained by modifying the solution clearance rate out of the stomach through adding muco-adhesive and/or viscosity builder polymers to the inventive composition.

Further advantages and features of the present invention will become apparent studying the following detailed description of preferred embodiments with reference to the accompanied drawings, wherein
Fig. 1 illustrates the percentage of diclofenac sodium being free in chitosan diclofenac sodium solution using chitosan of a fraction at pH 5.00;
Fig. 2 illustrates the percentage of diclofenac sodium being free in chitosan diclofenac sodium solution using chitosan of a fraction at pH 6.00;
Fig. 3 illustrates the percentage of diclofenac sodium being free in chitosan diclofenac sodium solution using chitosan of a fraction at pH 7.00; and
Fig. 4 illustrates the percentage of diclofenac sodium, being free in chitosan diclofenac sodium solution using chitosan of a fraction at pH 7.50;
Fig. 5 illustrates ibuprofen sodium plasma concentration time profiles of sustained release solution (molecular weight of chitosan < 3000) compared to an immediate release solution;
Fig. 6 illustrates ibuprofen sodium plasma concentration time profiles of sustained release solution (with chitosan having a molecular weight < 5000) compared to immediate release solution; and
Fig. 7 illustrates ibuprofen sodium plasma concentration time profiles of sustained release solution (chitosan having a molecular weight of < 10000) compared to immediate release solution.

### Examples

### Separation method of chitosan oligosaccharide

In order to prepare a liquid composition for sustained drug release according to the present invention, a chitosan fraction (or several fractions) have to be prepared from commercially available chitosan mixture.
A) 25 g chitosan oligosaccharide mixture was dissolved in 500 ml distilled water or 0.1 M HCl and adjusted to a pH of 1.0 using 6.0 M HCl. The chitosan oligosaccharide mixture may be a mixture comprising chitosan with a molecular weight of not more than 2,000, not more than 3,000, not more than 5,000, not more than 10,000, not more than 50,000, between 10,000 and 100,000, not more than 100,000 or 250,000-400,000, for example. Each chitosan solution was then filtered using a 0.45 µm cellulose acetate filter.
B) For each chitosan solution prepared above (having each a chitosan with different molecular weight) precipitates were collected at different pH values as follows:
   1. adjust pH of the filtered solution to pH 3.0 using 1.0 M sodium hydroxide (NaOH), filtrate and collect the precipitate,
   2. adjust pH of the filtered solution obtained in step 1 to pH 4.0 using 1.0 M sodium hydroxide (NaOH), filtrate and collect the precipitate,
   3. adjust pH of the filtered solution obtained in step 2 to pH 5.0 using 1.0 M NaOH, filtrate and collect the precipitate,
   4. adjust pH of the filtered solution obtained in step 3 to pH 5.5 using 1.0 M NaOH, filtrate and collect the precipitate,
   5. adjust pH of the filtered solution obtained in step 4 to pH 6.0 using 1.0 M NaOH, filtrate and collect the precipitate,
   6. adjust pH of the filtered solution obtained in step 5 to pH 6.5 using 1.0 M NaOH, filtrate and collect the precipitate,
   7. adjust pH of the filtered solution obtained in step 6 to pH 7.0 using 1.0 M NaOH, filtrate and collect the precipitate, and
   8. adjust pH of the filtered solution obtained in step 7 to pH 7.5 using 1.0 M NaOH, filtrate and collect the precipitate.
C) Above precipitates were purified by dissolving each of them in 0.1 M HCl. Then 1 M NaOH was added to increase the pH to the pH for precipitating each prepared solution. The precipitate was filtrated and collected. This step was repeated twice.
D) The above prepared precipitates were air-dried, milled and collected as powder.

Some of the chitosan precipitates obtained above in a pH range of 5.0 to 8.0 with chitosans having different molecular weights were used for further evaluation, and solutions for sustained drug release were prepared using that chitosan fractions and different drugs.

### 1. In-vitro drug release tests utilizing diclofenac sodium as a model drug

### Example 1

A precipitate collected in step B3 from chitosan mixture (molecular weight = not more than 100,000) at pH 5.0 was air-dried, milled and collected as powder. 2.0 g of this powder was dissolved in 20 ml of 0.1 M HCl. The pH of the solution was adjusted to 2.0 using 6.0 M HCl. The resultant concentration of chitosan is 10 % w/v.

### Further, the following solutions were prepared which are to be used for further evaluation:

Solution (1): 1.667 ml of 15 mg/ml diclofenac sodium in propylene glycol solution was added to 20 ml of the chitosan oligosaccharide solution prepared above.

Solution (2): 1.667 ml of 15 mg/ml of diclofenac sodium in propylene glycol solution was added to 20 ml of distilled water.

Solution (3): 1.667 ml of distilled water was added to 20 ml of the chitosan oligosaccharide solution prepared above [used as blank for solution 1].

Solution (4): 0.1 M HC1 solution was used and its pH was changed similar to the other solution [used as blank for solution 2].

The four solutions prepared were each added to 600 ml 0.1 M HCl and stirred with an USP paddle apparatus at a speed of 50 rpm at a temperature of 37°C. 0.2 M tri-sodium phosphate was added stepwise to slowly increase the pH value as follows:
1. 600 ml of 0.1 M HCl (pH 1.20).
2. Add to the above solution 105.0 ml of 0.2 M tri-sodium phosphate to get pH 2.92.
3. Add another 6.0 ml of 0.2 M tri-sodium phosphate to get pH 4.25.
4. Add another 3.0 ml of 0.2 M tri-sodium phosphate to get pH 5.20.
5. Add another 6.75 ml of 0.2 M tri-sodium phosphate to get pH 6.06.

The percentage of drug release from the chitosan fraction prepared according to step B3 (molecular weight not more than 100,000) is summarized as below. The drug release is measured by standard dissolution method, which is known to someone skilled in the art, see e.g., M. Sheu, H. Chou, C. Kao, C. Liu, T. Sokotoski, Dissolution of Diclofenac Sodium from Matrix Tablets, Int. J. Pharm., 85, pp. 57 - 63, 1992.

**Table 1**

| **pH** | **Absorbance of Solution (2)** | **Absorbance of Solution (1)** | **% Release** |
|---|---|---|---|
| 1.20 | 0.4078 | 0.1028 | 25.21 % |
| 2.92 | 0.5937 | 0.0902 | 15.20% |
| 4.25 | 0.7812 | 0.1506 | 19.28% |
| 5.20 | 1.0005 | 0.9810 | 98.05% |

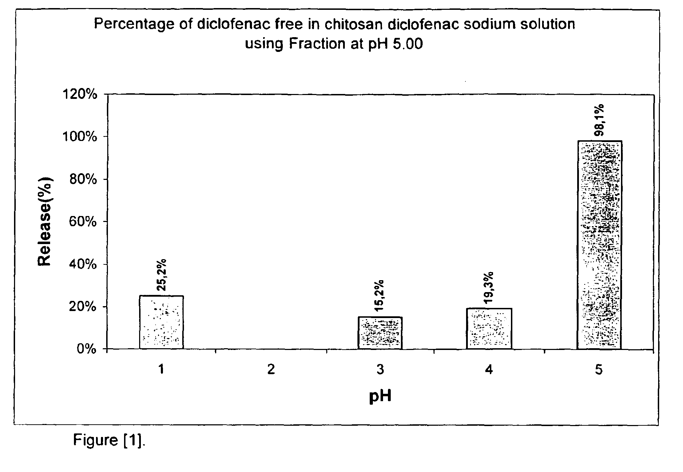

Fig. 1 illustrates the results given in table 1.

### Example 2

A precipitate collected in step B5 from chitosan oligosaccharide mixture (molecular weight = 10,000-100,000) at pH 6.0 was air-dried, milled and collected as powder. A chitosan solution was prepared as in example 1 above, and additionally four solutions as in example 1 were prepared. Further, the pH was increased by adding tri-sodium phosphate according to the following scheme:
1. 600 ml of 0.1 M HCl (pH 1.20).
2. Add to the above solution 105.0 ml of 0.2 M tri-sodium phosphate to get pH 2.92.
3. Add another 6.0 ml of 0.2 M tri-sodium phosphate to get pH 4.25.
4. Add another 3.0 ml of 0.2 M tri-sodium phosphate to get pH 5.20.
5. Add another 6.75 ml of 0.2 M tri-sodium phosphate to get pH 6.06.
6. Add another 45.0 ml of 0.2 M tri-sodium phosphate to get pH 6.88.

The percentage of drug release from the chitosan fraction obtained in step B5 (molecular weight = 10,000-100,000) is summarized as below.

**Table 2**

| **pH** | **Absorbance of Solution (2)** | **Absorbance of Solution (1)** | **% Release** |
|---|---|---|---|
| 1.20 | 0.4078 | 0.1228 | 30,01 % |
| 2.92 | 0.5937 | 0.1130 | 19,04% |
| 4.25 | 0.7812 | 0.2223 | 28,45% |
| 5.20 | 1.0005 | 0.9461 | 94,56% |
| 6.06 | 1.1108 | 1.0982 | 98.87% |

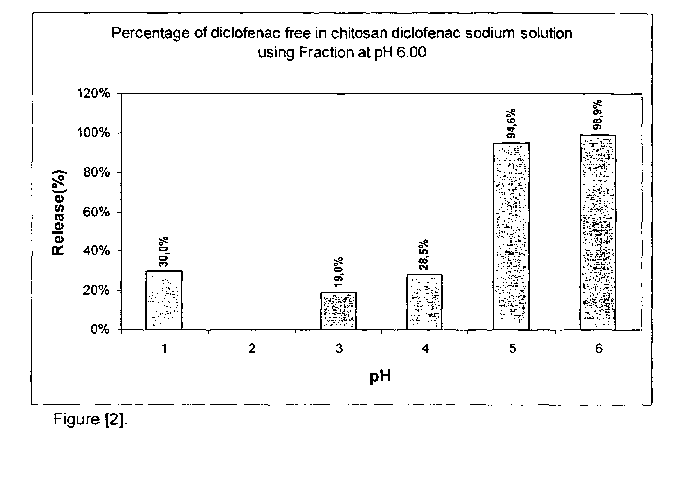

The results given in table 2 are also illustrated in Fig. 2

### Example 3

A precipitate collected in step B7 from chitosan oligosaccharide mixture (molecular weight = not more than 10,000) at pH 7.0 was air-dried, milled and collected as powder. A chitosan solution as well as solutions for evaluating the dissolution behavior were prepared according to example 1 and the pH of these solutions was amended using tri-sodium phosphate according to the following scheme.
1. 600 ml of 0.1 M HCl (pH 1.20).
2. Add to the above solution 105.0 ml of 0.2 M tri-sodium phosphate to get pH 2.92.
3. Add another 6.0 ml of 0.2 M tri-sodium phosphate to get pH 4.25.
4. Add another 3.0 ml of 0.2 M tri-sodium phosphate to get pH 5.20.
5. Add another 6.75 ml of 0.2 M tri-sodium phosphate to get pH 6.06.

The percentage of drug release from the chitosan fraction obtained in step B7 (molecular weight not more than 10,000) is summarized as below.

**Table 3**

| **pH** | **Absorbance of Solution (2)** | **Absorbance of Solution (1)** | **% Release** |
|---|---|---|---|
| 1,0 | 0.4078 | 0.0560 | 13.73% |
| 3,0 | 0.5937 | 0.0620 | 10.44% |
| 4,0 | 0.7812 | 0.0707 | 9.05% |
| 5,0 | 1.0005 | 0.2195 | 21.93% |
| 6,0 | 1.1108 | 1.0724 | 96.54% |

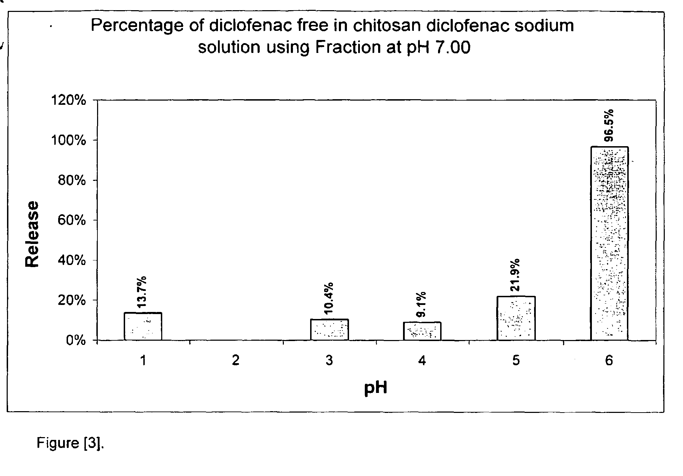

The results given in table 3 are also illustrated in Fig. 3.

### Example 4

A precipitate collected in step B8 from chitosan oligosaccharide mixture (molecular weight = not more than 5,000) at pH 7.5 was air-dried, milled and collected as powder. A chitosan solution as well as solutions for evaluating the dissolution behavior have been prepared according to example 1. The pH of these solutions was increased using tri-sodium phosphate according to the following scheme.
1. 600 ml of 0.1 M HCl (pH 1.20).
2. Add to the above solution 105.0 ml of 0.2 M tri-sodium phosphate to get pH 2.92.
3. Add another 6.0 ml of 0.2 M tri-sodium phosphate to get pH 4.25.
4. Add another 3.0 ml of 0.2 M tri-sodium phosphate to get pH 5.20.
5. Add another 6.75 ml of 0.2 M tri-sodium phosphate to get pH 6.06.
6. Add another 45.0 ml of 0.2 M tri-sodium phosphate to get pH 6.88.

The percentage of drug release from the chitosan fraction obtained in step B8 (molecular weight not more than 5,000) is summarized as below.

**Table 4**

| **pH** | **Absorbance of Solution (2)** | **Absorbance of Solution (1)** | **% Release** |
|---|---|---|---|
| 1.0 | 0.4078 | 0.065 | 15.84% |
| 3.0 | 0.5937 | 0.030 | 5.05% |
| 4.0 | 0.7812 | 0.073 | 9.40% |
| 5.0 | 1.0005 | 0.068 | 6.77% |
| 6.0 | 1.1108 | 0.556 | 50.08% |
| 7.0 | 1.0721 | 0.924 | 86.21% |
| 8.0 | 1.0204 | 0.990 | 97.04% |

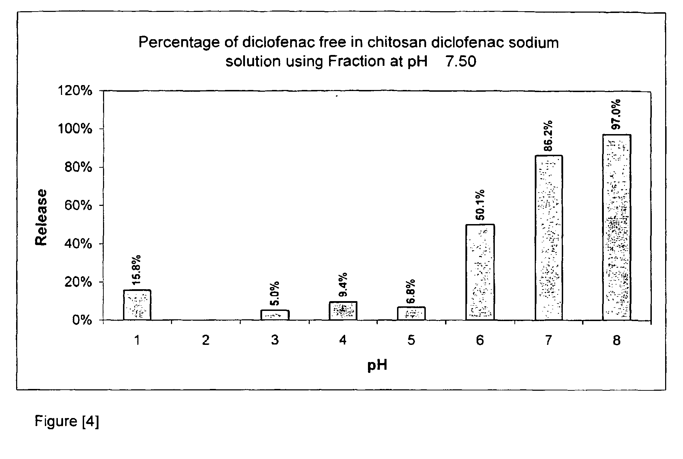

The results of table 4 are also illustrated in Fig. 4.

Above results illustrate that diclofenac sodium shows sustained release as pH is changing using different fractions of chitosan solutions. These situations resemble the gastrointestinal tract conditions when human being takes a solution orally.

The amount of the drug loaded in chitosan solution was 25 mg diclofenac sodium/22 ml. This dose seems realistic since the commercial diclofenac sodium doses start from 12.5 mg to 150 mg.

### 2. In vivo study on ibuprofen sodium chitosan conjugate (example not forming part of the invention)

Ibuprofen sodium is another model drug that is used to prove the sustained release idea in-vivo.

Chitosan of different molecular weights (Mw <3000, < 5000 and < 10000) were evaluated for controlling the release of ibuprofen. Each sustained release chitosan conjugate solution contained a complex of ibuprofen sodium with chitosan. The final pH of the solutions were 6.60. The dose of the conjugate oral solutions was 30 mg of ibuprofen/kg rabbit. A reference immediate release oral solution was given with a dose of 10 mg of ibuprofen/kg rabbit. Blood samples were withdrawn at specified time interval, centrifuged at 3500 rpm for 10 minutes and placed in a freezer at -20 °C before analysis. The samples were analyzed using HPLC method. A simple rapid method of determining the ibuprofen concentration by HPLC was developed. Naproxen was used as an internal standard. *Mobile Phase:* Water: Acetonitrile (40:60), then adjust pH to 2.4 with H3PO4. *Column:* Waters, Symmetry, 5u, C18, and 150*4.6 mm. *Internal STD Stock Solution:* Dissolve 5 mg of Naproxen into 50 ml MeOH. *Injection Loop:* 50ul. *Flow rate:* 1 ml/min. *Wavelength:* 220 nm.

Sample Preparation: Transfer 100 µL of plasma sample to test tube, add 10 µL of Internal STD Stock solution, add 0.25 ml of 1 M HCl, shake for 30 seconds, add 5 mL of (85:15)(Hexane: Isopropanol), shake with vortex for 1 min., centrifuge at 3000 rpm for 10 min., transfer 4 ml of organic layer to new test tube, evaporate the organic solvent using an air shower, and reconstitute with 1 ml mobile phase. The method was evaluated for specificity showing that there is no interference with the ibuprofen peak. Recovery was 85-90% for ibuprofen. The calibration curve was linear over the concentration 0.5-10 µg/ml.

The chitosan conjugate solution contained 12 mg ibuprofen/ml. The dose of the drug given to each rabbit was 30 mg/kg.

The immediate release aqueous solution contained ibuprofen sodium with a concentration of 4 mg ibuprofen/ml. The dose of the drug given as a reference was 10 mg/kg. This dose (10 mg/kg rabbit) is equivalent to 600 mg ibuprofen given to human subject weighted 60 kg (The usual human immediate release dose for adults is 200-600mg given 3 times per day). In the sustained release preparation the formula should contain total daily dose and to be given once i.e. 30 mg/kg.

This justifies the dose of the sustained release solution being three times more than the immediate release one. One of the advantages of sustained release is to decrease the frequency of drug dosing by giving the dose once per day.

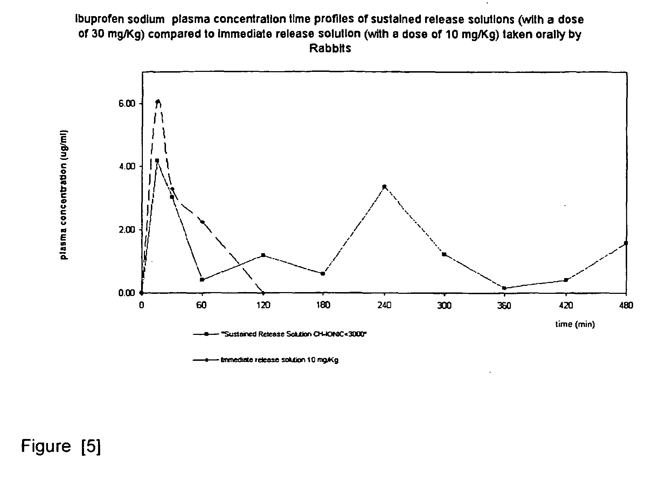

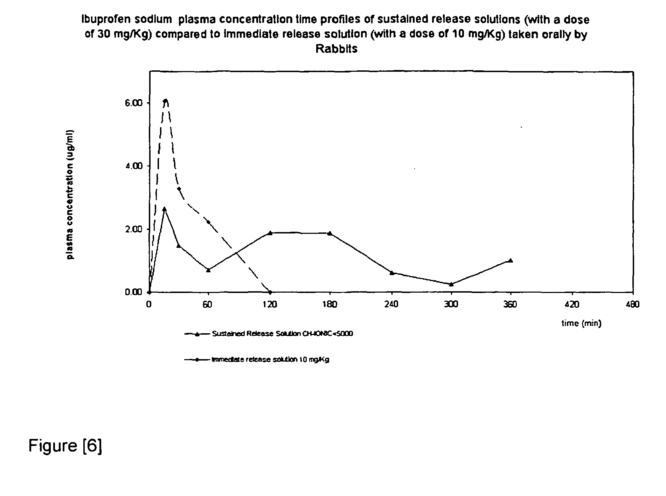

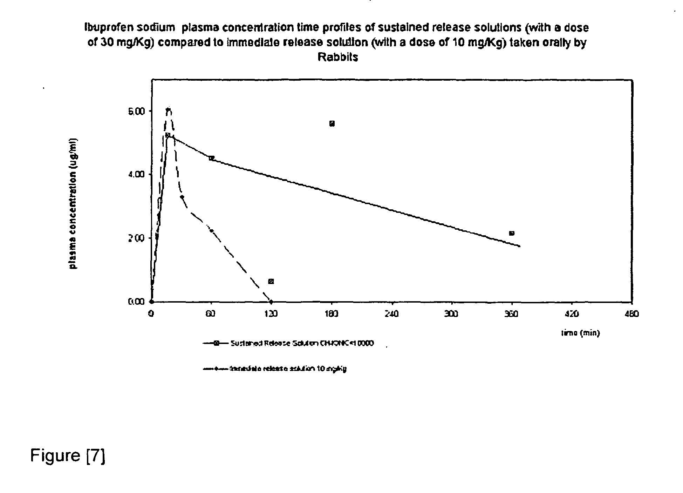

The drug release from the immediate release solution occurs very quickly, reaches maximum and also declines very quickly, Figures 5-7.

Figures 5-7 show that the release profiles of ibuprofen from the conjugate samples were pH dependent. As drug passes through a certain portion of different pH some of the drug is released. In the first portion, drug being released is somewhat equivalent or less than that released by an immediate release solution of ibuprofen.

For example, in the sustained release solution of CH MWt <3000 about 70 % of the immediate drug plasma level was reached in the first period based on Cmax ratio. While, in the sustained release solution of CH MWt <5000 and CH MWt <10000 about 45% and 86% of the immediate drug plasma level was reached in the first period, respectively. This clearly indicates that the 30 mg/kg sustained release dose was not absorbed rapidly. Small fractions of the drug were delivered as the complex passes throughout the GIT depending on the pH changes that occur inside the gut and the molecular weight of the chitosan conjugate with that drug.

Above results illustrate that ibuprofen shows sustained release as it passes the gastrointestinal tract when given as a chitosan conjugate solution.

The features disclosed in the foregoing description, in the claims and/or in the drawings may, both separately and in any combination thereof, be material for realizing the invention in diverse forms thereof.

## Claims

1. Use of a composition for preparation of a liquid sustained release delivery system for oral administration, wherein the composition comprises:
a) chitosan dissolved in a solvent having a pH of below 4.0, wherein the chitosan is selected from the group of chitosans precipitating in a pH range of between 5.0 and 7.5; and
b) at least one drug compound soluble in the above chitosan solution.

2. Use according to claim 1, wherein the chitosan is selected from the group of chitosans precipitating in a pH range of between 6.0 to 7.5, more preferably 6.5 to 7.5, and most preferably 7.0 and 7.5.

3. Use according to claim 1 or 2, wherein the solvent is 0.1 M HCl and/or water.

4. Use according to any of the preceding claims, wherein the drug is sodium diclofenac, ibuprofen sodium, valproate sodium and/or the like.

5. Use according to any of the preceding claims, wherein the drug is present in the composition in an amount of 0.1-10% w/v, preferably 0.1-2% w/v.

6. Use according to any of the preceding claims, wherein chitosan is present in the composition in an amount of 1-15% w/v, preferably 1-10% w/v, more preferably 1-5% w/v.

## Patentansprüche

1. Verwendung einer Zusammensetzung zur Herstellung eines flüssigen Abgabesystems mit nachhaltiger Freisetzung zur oralen Verabreichung, wobei die Zusammensetzung umfasst:
a) Chitosan, das in einem Lösungsmittel mit einem pH von unter 4,0 gelöst wird, wobei das Chitosan ausgewählt wird aus der Gruppe von Chitosanen, die in einem pH-Bereich zwischen 5,0 und 7,5 ausfallen;
b) wenigstens eine Wirkstoffverbindung, die in der obigen Chitosanlösung löslich ist.

2. Verwendung nach Anspruch 1, wobei das Chitosan ausgewählt wird aus der Gruppe von Chitosanen, die in einem pH-Bereich zwischen 6,0 bis 7,5, bevorzugter 6,5 bis 7,5 und am bevorzugtesten 7,0 bis 7,5 ausfallen.

3. Verwendung nach Anspruch 1 oder 2, wobei das Lösungsmittel 0,1 M HCl und/oder Wasser ist.

4. Verwendung nach einem der vorangehenden Ansprüche, wobei der Wirkstoff Natriumdiclofenac, Ibuprofen-Natrium, Valproat-Natrium und/oder dergleichen ist.

5. Verwendung nach einem der vorangehenden Ansprüche, wobei der Wirkstoff in der Zusammensetzung in einer Menge von 0,1-10 % w/v, bevorzugt 0,1-2% w/v vorliegt.

6. Verwendung nach einem der vorangehenden Ansprüche, wobei Chitosan in der Zusammensetzung in einer Menge von 1-15 % w/v, bevorzugt 1-10 % w/v, bevorzugter 1-5 % w/v vorliegt.

## Revendications

1. Utilisation d'une composition pour la préparation d'un système de distribution à libération soutenue d'un liquide, pour administration par voie orale, dans laquelle la composition comprend :
a) du chitosan dissous dans un solvant ayant un pH inférieur à 4,0, dans lequel le chitosan est choisi dans le groupe constitué de chitosans qui précipitent dans une gamme de pH comprise entre 5,0 et 7,5 ; et
b) au moins un composé de médicament soluble dans la solution de chitosan ci-dessus.

2. Utilisation selon la revendication 1, dans laquelle le chitosan est choisi dans le groupe de chitosans qui précipitent dans une gamme de pH comprise entre 6,0 et 7,5, de préférence 6,5 à 7,5, et de manière davantage préférée 7,0 et 7,5.

3. Utilisation selon les revendications 1 ou 2, dans laquelle le solvant est un 0,1 M HCl et/ou de l'eau.

4. Utilisation selon l'une quelconque des revendications précédentes, dans laquelle le médicament est du diclofénac de sodium, du sodium d'ibuprofène, du valproate de sodium, et/ou similaire.

5. Utilisation selon l'une quelconque des revendications précédentes, dans laquelle le médicament est présent dans la composition, dans une quantité de 0,1-10% en poids, de préférence de 0,1-2% en poids.

6. Utilisation selon les revendications précédentes, dans laquelle le chitosan est présent dans la composition, dans une quantité de 1-15% en poids, de préférence 1-10% en poids, de préférence 1-5% en poids.
